(19) Europäisches Patentamt
European Patent Office
Office européen des brevets

(11) Publication number: 0 095 813
A2

(12) # EUROPEAN PATENT APPLICATION

(21) Application number: 83200757.9

(22) Date of filing: 27.05.83

(51) Int. Cl.³: A 61 K 31/52, A 61 K 47/00

(30) Priority: 01.06.82 US 383891
06.05.83 US 489663

(43) Date of publication of application: 07.12.83
Bulletin 83/49

(84) Designated Contracting States: AT BE CH DE FR GB IT LI LU NL SE

(71) Applicant: THE PROCTER & GAMBLE COMPANY,
301 East Sixth Street, Cincinnati Ohio 45202 (US)

(72) Inventor: Cooper, Eugene Rex, 2424 Ambassador Drive, Cincinnati Ohio 45231 (US)

(74) Representative: Suslic, Lydia et al, Procter & Gamble European Technical Center Temselaan 100, B-1820 Strombeek-Bever (BE)

(54) **Penetrating topical pharmaceutical compositions containing 9-(2-hydroxyethoxymethyl) guanine.**

(57) A group of hydrophilic substituted purine antivirals, especially 9-(2-hydroxyethoxymethyl) guanine, can be effectively delivered across skin by incorporating the selected antiviral into a binary, penetration-enhancing vehicle containing (1) a $C_3$–$C_4$ diol, diol ester or diol ether, and (2) a cell-envelope disordering compound. These compositions provide surprising enhancement of delivery for the antivirals when compared to existing vehicles or systems, and provide a method of treating viral infections. They are especially effective as antiherpetics.

EP 0 095 813 A2

# PENETRATING TOPICAL PHARMACEUTICAL COMPOSITIONS CONTAINING 9-(2-HYDROXYETHOXYMETHYL) GUANINE

Eugene R. Cooper

## TECHNICAL FIELD

The present invention relates to a composition effective in making certain hydrophilic pharmaceutically-active agents bioavailable by delivering these agents through the integument.

Because of the ease of access, dynamics of application, large surface area, vast exposure to the circulatory and lymphatic networks, and non-invasive nature of the treatment, the delivery of pharmaceutically-active agents through the skin has long been a promising concept. This is true whether the bioavailability desired is systemic or dermal, regional or local.

The advantages of this form of delivery include, but are not limited to: avoidance of the risks associated with parenteral treatment; elimination of the inconveniences of parenteral treatment; avoidance of the variable rates of absorption and metabolism inherent in oral treatment; increasing the continuity of drug administration by permitting delivery of agents with short biological half-lives; and elimination of gastrointestinal irritation resulting from exposing the gastrointestinal tract to pharmaceutical actives, preservatives, tableting agents, and the like. Most importantly, topical delivery possesses the ability to effectively treat conditions which are local in nature, (or which exhibit local manifestations), systemically as well as locally with the same treatment regimen.

However, because it must serve as a barrier to the ingress of pathogens and toxic materials, and the egress of physiologic fluids, the skin is highly impermeable. It must be impermeable to preserve its own integrity while at the same time maintaining the delicate dynamic electrolyte balance of the body.

A good deal of this impermeability of the skin results from the nature of one very thin layer created by normal developmental and physiological changes. After cells are formed in the basal layer, they begin to migrate toward the surface, until they are eventually sloughed off. As they undergo this migration, they become progressively more keratinized. When they reach the surface, just prior to being discarded, they form a thin layer approximately ten microns thick. This layer is called the stratum corneum. As a result of the level of keratinization of the cells which comprise the stratum corneum, a formidable barrier is created. Therefore, penetration via the nonpolar route, i.e., across the cell membrane, remains most difficult.

Other possible penetration routes are available. First, any mechanism which allows the egress of materials, e.g. the sebaceous apparatus, can be manipulated to allow the ingress of materials. Second, the stratum corneum, though keratinized to a great degree, is composed of about 15% lipid-based intercellular material. This may offer a less formidable route despite the close packing of the cells.

It is known that certain binary skin penetration systems can increase the disorder of these lipids. By so increasing the disorder of the lipid portion of the cell-envelope in the stratum corneum, the lipid packing of the cells can be disrupted. This disruption allows certain lipid-soluble drugs to pass through the stratum corneum. This discovery has been confirmed by differential scanning calorimetery, indicating that certain binary skin penetration enhancement systems eliminate the Tm-2 peak associated with melting of cell-envelope lipids.

Herpes is a viral disease the occurrence of which is of epidemic proportions. Its spread is unchecked, partly due to lack of an antiviral which is effective in treating herpes topically.

Herpes is characterized by painful, and occasionally debilitating, cutaneous manifestations. These manifestations could be most effectively treated locally, i.e., by topical application of antivirals.

9-(2-hydroxyethoxymethyl) guanine, (also known as Acyclovir), is one of the few antivirals known to be effective in herpes treatment. However, this agent suffers from the disadvantage of being slightly soluble in water and almost totally insoluble in lipophilic solvent systems. Further, while 9-(2-hydroxyethoxymethyl) guanine is hydrophilic, conventional penetration-enhancing preparations designed for hydrophilic actives have been largely ineffective in delivering this antiviral through the skin. Attempts to design a penetration system or vehicle specifically for this active have met with limited success. See European Patent Application 44,543, published January 27, 1982, incorporated herein by reference.

Like most pharmaceutical actives, the effectiveness of 9-(2-hydroxyethoxymethyl) guanine as a topical therapeutic agent is a function of the ability of a vehicle to deliver it in bioavailable form. Accordingly, the inability to find an effective delivery system or vehicle has limited 9-(2-hydroxyethoxymethyl) guanine's effectiveness in a topical treatment regimen.

It has now surprisingly been discovered that certain binary penetration systems heretofore thought only to be effective for delivery of lipophilic pharmaceutical active agents can now be used to make the topical mode of treatment of hydrophilic agents practical by percutaneously delivering clinically effective doses of these hydrophilic drug actives. Specifically, it has been discovered that the binary penetration system comprising a cell-envelope disordering compound and a diol compound, heretofore thought only to be useful in delivering lipophilic actives, can dramatically improve the topical delivery of certain hydrophilic antivirals, such as 9-(2-hydroxyethoxymethyl) guanine, and related compounds.

U.S. Patent Application, Serial Number 001,974, Wickett, et al., filed January 8, 1979, describes compositions useful in the treatment of acne. These compositions contain benzoyl peroxide, $C_6$ - $C_{14}$ primary alcohols, and a diol selected from 1,2-propanediol, 1,2-butanediol, 1,3-butanediol, and 2,3-butanediol. A

foreign equivalent of this application was made available to the public July 23, 1980. See European Patent Application 13,459.

U.S. Patent Application Serial Number 296,706, Cooper, et al., filed August 27, 1981, describes compositions for topical application. These compositions described are suitable for effective delivery of lipophilic, pharmacologically active compounds using primary alcohols or various carboxylate compounds in combination with selected diols. See European Patent Application 43,738.

U.S. Patent 4,199,475, Schaeffer, April 22, 1980, discloses and claims 9-hydroxyethoxymethyl (and related) derivatives of 6- and 2,6-substituted purines. These compounds are reported to be effective in the treatment of viral infection, especially herpes, and can be administered parenterally, orally or topically. 9-(2-hydroxyethoxymethyl) guanine is disclosed as being particularly active.

U.S. Patent 4,146,715, Schaeffer, March 27, 1979, discloses a method of preparing 9-(2-hydroxyethoxymethyl) guanine.

European Patent Application 44,543, published January 27, 1982, describes topical formulations containing 9-(2-hydroxyethoxymethyl) guanine. This application indicates that optimum release of the active occurs when it is solubilized in the aqueous phase of an oil-in-water emulsion.

1,2-propanediol ("propylene glycol") and the $C_{10}$-$C_{14}$ alcohols have been used, separately, in cosmetic and pharmaceutical formulations. In particular, propylene glycol has been described in several articles in the literature as enhancing the penetration of certain pharmacologically active agents, such as the corticosteroids.

U.S. Patent 3,535,422, Cox, et al., October 20, 1970, relates to stable benzoyl peroxide compositions containing organic emollients. The compositions include emollients selected from the $C_4$-$C_{20}$ aliphatic alcohols, $C_2$-$C_3$ glycols, $C_{12}$-$C_{20}$ fatty acids and their esters, and mixtures thereof.

U.S. Patent 4,070,462, Ecker, issued January 24, 1978, describes topical steroid compositions containing 6% propylene glycol and 1% propylene glycol monostearate.

Canadian Patent 1,072,009, describes topical antimicrobial compositions containing $C_5$-$C_{10}$ straight chain alcohols or $C_{17}$ branched chain alcohols in which the longest chain is $C_5$-$C_{10}$.

CA 92:153,181j; describes an indomethacin ointment containing 10% propylene glycol and 1.1% diisopropanolamine.

U.S. Patent 2,990,331, Neumann, et al., issued June 27, 1961, describes tetracycline compositions containing carboxylic acid alkylolamides.

H. Barnes, et al., Br. J. Derm. 93, 459 (1975) describe testing of fluocinonide and fluocinolone acetonide in a vehicle described as fatty alcohol propylene glycol (FAPG).

P.J.W. Ayres, et al., Br. J. Derm., 99, 307 (1978), report comparative skin penetration of cortisol from commercially available cortisol ointments.

Schaaf and Gross, Dermatologica, 106, 357 (1953) note that unsaturated fatty acids and $C_6$-$C_{14}$ saturated fatty acids are particularly active in provoking epidermal thickening.

J. Zatz, et al., J. Pharm. Sci., 67, 789 (1978) describe the effect of formulation factors on penetration of hydrocortisone through mouse skin.

S. K. Chandrasekaran, et al., J. Pharm. Sci., 67, 1370 (1978) discuss the pharmacokinetics of drug permeation through human skin.

B. Idson, Cosmetics & Toiletries, 95, 59 (1980), states the factors affecting drug penetration and, consequently, in most cases effectiveness, are complex. He observes that the vehicle that provides ideal conditions for one drug may prove unsatisfactory for another. The author concludes that prediction is not simple and product suitability must be assessed by human trials. The same article indicates that Synalar Cream, a topical corticosteroid preparation, contains sorbitan monooleate and propylene glycol.

M. M. Rieger, Cosmetics & Toiletries, 94, 32-37 (1979) and 95, 26-38 (1980), provides a review of current literature in the area of skin penetration.

U.S. Patent 4,299,826, Luedders, November 10, 1981, describes a composition for the treatment of acne by using diisopropyl sebacate as a penetration enhancer for an erythromycin derivative in combination with an alcohol.

U.S. Patent 2,990,331, Neumann, et al., describes the parenteral administration of tetracycline salts from a stable aqueous solution.

CA 79: 122,308, describes an electromagnetic study of n-alkyl ionic surfactants as aiding in human epidermis penetration.

## SUMMARY OF THE INVENTION

The present invention encompasses compositions and methods for topically treating herpes and other viral infections in animal tissue, especially in humans. The invention provides effective penetration compositions and therapy, and is based on the use of a hydrophilic antiviral together with a binary mixture of a cell-envelope disordering compound and a diol compound.

The compositions of this invention comprise a safe and effective amount of a 6- or 2,6- substituted purine antiviral active, particularly 9-(2-hydroxyethoxymethyl) guanine, together with a penetration-enhancing vehicle containing a $C_3$-$C_4$ diol and a cell-envelope disordering compound, such as oleic acid. The composition acts to provide effective topical treatment by efficiently delivering the antiviral active, in bioavailable form, through the skin. The effectiveness of this binary mixture, designed for lipophilic actives, is surprising in light of the hydrophilic nature of the active.

The invention also encompasses treatment regimens for viral infections comprising topically administering to a human or lower animal in need of such treatment a safe and effective amount of these compositions at the afflicted situs.

## DESCRIPTION OF THE INVENTION

The compositions of this invention require, at a minimum, a hydrophilic pharmaceutical active effective against viral infection, a diol compound, and a cell-envelope disordering compound. Compositions of this invention may additionally contain other optional components which reduce skin irritation, or enhance their cosmetic appeal and acceptability, i.e., thickeners, pigments, and the like.

## CELL-ENVELOPE DISORDERING COMPOUND

The cell-envelope disordering compounds can be described by the formula $R^1$-X, where $R^1$ is a straight chain alkyl of about 7 to about 16 carbon atoms, a branched-chain alkyl of about 13 to about 22 carbon atoms, or an alkenyl of about 7 to about 22 carbon atoms. When an alkenyl with a single double bond is selected as $R^1$ in the cell-envelope disordering compound, it cannot have the sole double bond in the terminal position (a terminal position double bond in an alkenyl reduces its ability to aid in disordering the cell-envelope). Such compounds are therefore referred to as "nonterminal alkenyls." X in this formula is

$-OH$, $-COOH$, $-COOR^2$, or $-O-\overset{O}{\overset{\|}{C}}-R^3$, where $R^2$ is $-CH_3$, $-C_2H_5$, $-C_3H_7$, or $-C_2H_4OH$, $R^3$ is $-H$, $-CH_3$, $-C_2H_5$, $-C_3H_7$, $-C_4H_9$; X may also be

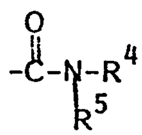

where $R^4$ is $-H$, $-CH_3$, $-C_2H_5$, $-C_3H_7$ or $-C_2H_4OH$, $R^5$ is $-H$, $-CH_3$, $-C_2H_5$, $-C_3H_7$ or $-C_2H_4OH$; X may also be $-SOCH_3$, $-P(OCH_3)_2O$, $-COOC_2H_4OC_2H_4OH$, $-COOCH_2(CHOH)_4CH_2OH$, $-(OCH_2CH_2)_mOH$, where m is an integer from 2 to 6.

Alkenols and alkenoic acids with a single double bond must be in the cis-configuration. This requirement does not apply, however, to the methyl sulfoxides, phosphine oxides, propylene glycol esters or glycerol esters, possibly because these head groups, while fairly short, are comparatively bulky, requiring greater spacing of the molecules, and thus causing additional

disruption of the orderly lipid structure of the cell-envelope.

Compounds of the foregoing category, which disrupt or disorder the lipid structure of stratum corneum cell-envelopes, include, without limitation, the n-octyl, decyl, dodecyl, and tetradecyl alcohols; the methyl and ethyl esters of n-octanoic, decanoic, dodecanoic, tetradecanoic and hexadecanoic acid; the acetates of n-octanol, decanol, dodecanol, tetradecanol and hexadecanol, such as lauryl acetate and myristyl acetate; the methyl, ethyl, N,N-dimethyl, -diethyl and -methylethyl amides of octanoic, decanoic, dodecanoic, tetradecanoic and hexadecanoic acids; the decenyl, dodecenyl, tetradecenyl, hexadecenyl, octadecenyl, eicosenyl, and docosenyl alcohols in which the double bond has the cis-configuration; cis-myristoleic, palmitoleic, oleic, linoleic, linolenic, arachidonic, and erucic acids; tetradecenyl methyl sulfoxide, hexadecenyl methyl sulfoxide, octadecenyl methyl sulfoxide, eicosenyl methyl sulfoxide, and docosenyl methyl sulfoxide; methyl cis-myristoleate, -palmitoleate, -oleate, -linoleate, -linolenate, -arachidonate, and -erucate; oleic isopropyl amide, dimethyl glycol oleate, polyethylene glycol 200 monolinoleate, dioxyethylene isostearyl ether, dioxyethylene oleyl ether, sorbitan monoleate, sorbitan monoisostearate, and ethyl isostearate; myristoleyl, palmitoleyl, oleyl, linoleyl, linolenyl, arachidonyl, and erucyl dimethyl phosphine oxides; isohexadecyl dimethyl phosphine oxide, isooctadecyl dimethyl phosphine oxide, isoeicosenyl dimethyl phosphine oxide, and isodocosyl dimethyl phosphine oxide; isohexadecyl monoethanolamide, isooctadecyl monoethanolamide, isoeicosyl monoethanolamide, and isodocosyl monoethanolamide; propylene glycol monoisopalmitate, propylene glycol monoisostearate, propylene glycol monoisoarchidate, propylene glycol monoisobehenate, glycerol monoisopalmitate, glycerol monoisostearate, glycerol monoisoarachidate, and glycerol monoisobehenate; and phytol, bactoprenol, geraniol, isophytol, and farnesol. Mixtures of the above may also be used.

Preferred cell-envelope disordering compounds include oleic, decenoic, dodecenoic, tetradecnoic, hexadecenoic, heptadecenoic, eicosenoic, and docosenoic acids; oleyl, decenyl, dodecenyl,

tetradecanyl, hexadecanyl, heptadecanyl, eicosenyl, and docosenyl alcohols; the methyl, ethyl, propyl and isopropyl esters of decanoic, dodecanoic, and tetradecanoic acids; and the acetates of n-octanol, decanol, dodecanol, and tetradecanol. The most preferred cell-envelope disordering compounds include oleic acid, myristyl alcohol, methyl myristate, methyl laurate, ethyl myristate, ethyl laurate, myristyl acetate and lauryl acetate. Mixtures of the above may also be used.

DIOL COMPOUNDS

The diol compounds which may be used this invention include diols, diol ethers, and diol esters. They can be represented by the general formula (I)

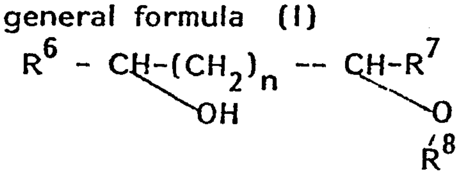

(I)

where $R^6$ is -H, $-CH_3$ or $-CH_2O\overset{O}{\overset{\|}{C}}CH_3$, $R^7$ is -H or $-CH_3$, $R^8$ is H, $-C_2H_5$ or $-CH_2H_4OH$, and n is 0 or 1.

Examples of diols of the foregoing formula include, without limitation, 1,2-ethanediol (ethylene glycol), 1,2-propanediol, 1,3-propanediol, 1,2-butanediol, 1,3,-butanediol, 1,4-butanediol, 2,3-butanediol, 2-ethoxyethanol (ethylene glycol monoethyl ether), 2,2 oxy-bis-ethanol (diethylene glycol) and glycerol monoacetate. Mixtures may also be used.

Preferred diols include 1,2-propanediol and 2,2-oxy-bis-ethanol.

Binary mixtures of any of the foregoing diol compounds and cell-envelope disordering compounds, in a weight ratio of diol compound:cell-envelope disordering compound of from about 1:1 to about 500:1 provide significant enhancement of penetration for the hydrophilic antiviral agents described herein. A ratio of diol compound:cell-envelope disordering compound of from about 5:1 to about 100:1 is preferred, with a ratio of about 10:1 to about 100:1 being more preferred. The penetrating components are most preferably present in a ratio of about 10:1 to about 50:1.

The compositions useful in this invention typically contain from about 0.01% to about 99%, and preferably about 50% to about 99%, by weight, of the penetration enhancing binary mixture of the diol compounds and cell-envelope disordering compounds, employing the ratios described above.

## ANTIVIRAL ACTIVES

Antiviral compounds used in this invention are of the formula (II)

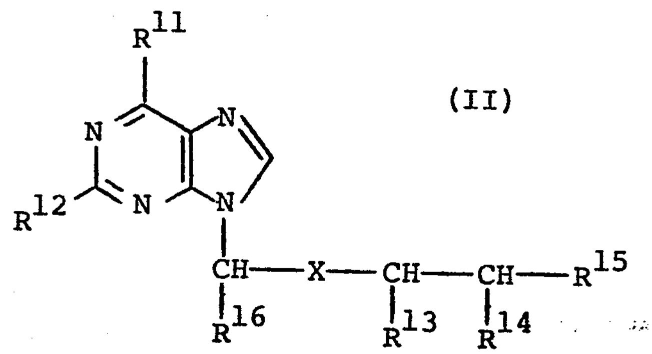

(II)

wherein X is sulfur or oxygen, $R^{11}$ is hydrogen, a halogen, hydroxy, alkoxy, azide, thio, alkylthio, amino, alkylamino or dialkylamino; $R^{12}$ is hydrogen, a halogen, alkylthio, acylamino, amino or azide; $R^{13}$ is hydrogen, a straight, branched or cyclic alkyl, hydroxyalkyl, benzyloxyalkyl or phenyl, $R^{14}$ is hydrogen, hydroxy or alkyl; $R^{15}$ is hydrogen, hydroxy, amino, alkyl, hydroxyalkyl, benzyloxy, benzoyloxy, benzoyloxymethyl, sulfamoyloxy, phosphate carboxypropionyloxy, straight chain or cyclic acyloxyl having from 1 to 8 carbon atoms, or substituted carbamoyl group having the formula NHCO-Z; wherein Z is alkyl, aryl, or aralkyl optionally substituted by one or more substituents selected from sulfonyl, amino, carbamoyl or hydrogen; $R^{16}$ is hydrogen or an alkyl, provided that when X is oxygen and $R^{12}$, $R^{13}$, $R^{14}$ and $R^{16}$ are H, $R^{11}$ is not amino or methylamino when $R^{15}$ is hydrogen or hydroxy. Pharmaceutically acceptable organic and inorganic acid salts of the above, such as the salt of lactic,

acetic, malic, hydrochloric and sulfuric acids, may also be employed. The reaction product of the above with zinc or zinc salts may also be employed.

Preferred antiviral compounds of this invention are of the formula (II), above, wherein X is sulfur or oxygen, $R^{11}$ is hydroxy, $R^{12}$ is amino, $R^{13}$ is hydrogen, a straight or branched chain or cyclic alkyl, hydroxyalkyl, benzyloxyalkyl or phenyl, $R^{14}$ is hydrogen, hydroxy or lower alkyl, $R^{15}$ is hydrogen, hydroxy amino, alkyl, hydroxyalkyl, benzoyloxy, benzoyloxy alkyl, benzyloxy, sulfamoyloxy, phosphate, carboxypropionyloxy, or acetoxy, $R^{16}$ is hydrogen, alkyl or hydroxyalkyl, or a pharmaceutically-acceptable salts of the above.

Highly preferred antiviral compounds of this invention include 9-(2-hydroxyethoxymethyl) guanine, 2-amino-9-(2-hydroxyethoxymethyl) adenine, and pharmaceutically-acceptable salts, and pharmaceutically-acceptable esters, of these compounds. Especially preferred are 9-(2-hydroxyethoxymethyl) guanine, as well as its pharmaceutically-acceptable salts and esters.

The method of preparing these compounds, and their uses and clinical indications as antivirals, are well-known to those skilled in the art. See U.S. Patent 4,199,574, Schaeffer, issued April 22, 1980, herein incorporated by reference.

The compositions of this invention typically contain from about 0.01 to about 10% by weight of the substituted purine antiviral, and preferably contain about 0.05% to about 5%.

OPTIONAL COMPONENTS

In addition to the components described above, the compositions of this invention may optionally contain a cosmetically acceptable solvent. The solvent, if used, should not significantly interfere with the penetration action of the binary combination of the diol compound and cell-envelope disordering compound, and should preferably evaporate rapidly and completely to leave only the active components of the composition at the site of application. Preferred solvents include ethanol and isoproponol.

Such solvents may comprise from 0 to about 70% of the total composition.

Water may be used in the compositions of the present invention. However, simple addition of water to these compositions may cause the cell-envelope disordering compound to precipitate out. This action could significantly reduce the overall effectiveness of the cell-envelope disordering compound. In order to prevent this, it is preferred that if water is used, an emulsion must be formed to disperse the cell-envelope disordering compound within the composition. Since these compounds themselves, used alone, do not form an emulsion stable enough for the intended use of the compositions, hydrophilic emulgents must therefore be employed. However, hydrophilic emulgents significantly interfere with the efficacy of the compositions. Therefore, because of the need for these emulgents, the use of emulsions should be avoided in formulating compositions of the present invention, and accordingly, the amount of water used in the compositions of the present invention is preferably kept at relatively low levels. Therefore, the preferred compositions of the present invention comprise from 0% to about 15%, and more preferably comprise less than 10% water, by weight. The compositions are most preferably substantially water-free, i.e., contain less than 1% water.

Other optional components can be added to the formulations of this invention to enhance their cosmetic acceptability. Such components include thickening agents, such as methyl cellulose, cross-linked carboxypolymethylene polymers, bentonite, gum tragacanth, gum karaya, and the like. Thickeners are generally employed at a level of from about 1% to about 10% of the compositions.

Minor amounts of other materials may be added at art established levels. These include, without limitation, pigments opacifiers, fragrances, perfumes, and the like. Such materials, when added, should not unduly interfere with the penetration enhancement of these compositions. Such formula modifications to improve cosmetic acceptability are well within the skill of workers in the cosmetic and dermatological arts and, by themselves, constitute no part of the present invention.

All optional components should be selected to prevent substantial interference with the penetration ability of the composition.

It can be seen from the foregoing that the compositions of the present invention admit of considerable variation, so long as the critical components of antiviral active, diol compound, and cell-envelope disordering compounds are present within the ranges indicated above.

METHOD OF USE

It will be appreciated that this invention provides a method for treating and preventing viral infections, and associated manifestations, in humans and lower animals, comprising topically applying to a human or lower animal in need of such treatment a safe and effective amount of a composition of this invention at the afflicted situs. It will also be appreciated that this invention provides a method for treating specific conditions caused by viral infections of the skin and mucosa, especially those caused by herpes simplex, herpes zoster, and herpes varicella.

The compositions of this invention are typically applied one to six times daily to the afflicted situs. When systemic effects are also desired, or when it is desired to reduce the chance of the spread of infection, the compositions of this invention are applied to larger areas.

A typical safe and effective usage rate is about 1 mg of the total topical composition per square centimeter of skin to about 10 mg of total topical composition per square centimeter of skin. The skilled artisan will appreciate that this application rate will vary with the condition being treated, its progress, the area it has involved, the severity of the infection, the nature of the actives, or carriers, the physical condition of the patient, concurrent therapies being administered and the concentration of the actives or carriers being used. However, usage rates of up to 500 mg of total composition per square centimeter of skin may be used when the composition is used as an occlusive dressing.

The compositions can be applied once every twenty-four hours to about twenty-four times every twenty-four hours.

Application intervals of every 4 hours to every 12 hours are preferred. A treatment regimen of application every 6 hours is particularly preferred because it minimizes the amount of antiviral which is applied at one time while reducing the inconvenience of frequent applications. However, any treatment regimen which allows a safe and effective amount of antiviral to reach the afflicted situs can be employed while using the compositions of this invention.

By "topical administration" herein is meant directly laying on or spreading on epidermal tissue, especially outer skin.

By "safe and effective amount" of the compositions herein is meant a sufficient amount of the composition to alleviate or prevent the viral infection or disease state being treated at a reasonable benefit/risk ratio attendant with any medical treatment. Within the scope of sound medical judgment, the amount of pharmaceutical active used will vary with the particular condition being treated, the severity of the condition, the duration of the treatment, the specific compound employed and its concentration, the condition of the patient, concurrent therapies being administered, and like factors within the specific knowledge and expertise of the patient or the attending physician.

By "toxicologically- or pharmaceutically- acceptable" herein is meant ingredients which are suitable for use in contact with the tissues of humans and lower animals without undue toxicity, irritation, allergic response, and the like, commensurate with a reasonable benefit/risk ratio.

By the term "comprising" herein is meant that various other compatible drugs and medicaments, as well as inert ingredients, occlusive agents, and cosmetic vehicles, can be conjointly employed in the compositions and processes of this invention, as long as the critical binary penetration enhancement vehicle and pharmaceutical active are used in the manner disclosed herein. The term "comprising" thus encompasses and includes the more restrictive terms "consisting of" and "consisting essentially of" which characterize the use of the essential ingredients in the manner disclosed herein.

By "afflicted situs" herein is meant a localized area of inflammation or lesion, and the immediately surrounding area.

By "penetration-enhancing" herein is meant that the binary penetration enhancing carriers of this composition provide marked transepidermal delivery of an incorporated pharmacological active, when compared to other compositions at equal chemical potential. This latter aspect is important, since varying solubilities of drugs in different vehicles will necessarily affect their transport across skin. Thus, for example, if a drug is soluble in vehicle A to the extent of 24%, and in vehicle B to the extent of 4%, if the compositions were to be compared at equal percentage concentration, rather than equal chemical potential, the lower solubility carrier will show a misleading six-fold difference in transport over the more soluble vehicle. The simplest way of assuring equal chemical potential for evaluating penetration enhancement is to use saturated solutions or solutions of equal percentage of saturation of pharmacological active in the various vehicles.

By "nonterminal alkenyl" herein is meant that the double bond is not found between the last two carbons in the hydrocarbon tail of the compound when the compound has a single double bond; terminal alkenyls are, from a structural standpoint, almost identical to fully saturated compounds, since the double bond, while rigid, does not affect the configuration of the chain.

By the "cis- configuration" herein is meant that both portions of the hydrocarbon chain are positioned on the same side of the double bond when the compound has a single double bond.

The following are nonlimiting examples of compositions the invention. All components are thoroughly mixed together to form the compositions.

EXAMPLE I

| | |
|---|---|
| 9-(2-hydroxyethoxymethyl)guanine | 1% |
| myristyl alcohol | 5% |
| diethylene glycol | 94% |

EXAMPLE II

| | |
|---|---|
| 9-(2-hydroxyethoxymethyl) guanine | 1% |
| oleyl alcohol | 5% |
| propylene glycol | 94% |

EXAMPLE III

| | |
|---|---|
| 9-(2-hydroxyethoxymethyl) guanine | 1% |
| oleic acid | 5% |
| propylene glycol | 94% |

EXAMPLE IV

| | |
|---|---|
| 9-(2-hydroxyethoxylmethyl) guanine | 1% |
| lauryl alcohol | 5% |
| diethylene glycol | 94% |

EXAMPLE V

| | |
|---|---|
| 9-(2-hydroxyethoxylmethyl) guanine | 0.5% |
| methyl laurate | 1% |
| 1,3-butanediol | 50% |
| ethanol | 48.5% |

EXAMPLE VI

| | |
|---|---|
| 9-(2-hydroxyethoxymethly) guanine | 0.5% |
| ethyl laurate | 1% |
| diethylene glycol | 50% |
| ethanol | 48.5% |

EXAMPLE VII

| | |
|---|---|
| 9-(2-hydroxyethoxymethyl) guanine | 0.5% |
| methyl laurate | 1% |
| propylene glycol | 50% |
| ethanol | 48.5% |

EXAMPLE VIII

| | |
|---|---|
| 9-(2-hydroxyethoxymethyl) guanine | 0.5% |
| lauryl acetate | 1% |
| propylene glycol | 50% |
| isopropyl alcohol | 48.5% |

EXAMPLE IX

| | |
|---|---|
| 2-amino-9-(2-hydroxyethoxymethyl) adenine | 0.5% |
| oleic acid | 1% |
| propylene glycol | 50% |
| ethanol | 48.5% |

EXAMPLE X

| | |
|---|---|
| 2-amino-9-(2-hydroxyethoxymethyl) adenine | 0.5% |
| oleic acid | 1% |
| 2,2-oxy-bis-ethanol | 50% |
| ethanol | 48.5% |

The following are nonlimiting examples of methods of treatment of the invention.

EXAMPLE XI

The composition of Example I is applied to a human afflicted with a herpetic lesion at the afflicted situs at a rate of 5 $mg/cm^2$ three times daily for a period of 2 weeks. Reduction in the size of the lesion is noted after 3 days.

EXAMPLE XII

The composition of Example II is applied to a human afflicted with a herpetic lesion at the afflicted situs at a rate of $1mg/cm^2$ six times daily for a period of 1 week. Reduction in the size of the lesion is noted after 3 days.

EXAMPLE XIII

SKIN PENETRATION STUDIES

The following data illustrate the effects of incorporating 9-(2-hydroxyethoxymethyl) guanine into a standard vehicle/solvent versus the same pharmaceutical active used in a binary system with a cell-envelope disordering compound.

| Vehicle | Relative Penetration Trial 1 | Trial 2 | Trial 3 |
|---|---|---|---|
| 0.25% 9-(2-hydroxyethoxymethyl) guanine<br>99.75% propylene glycol | 1 | 1 | 1 |
| 0.25% 9-(2-hydroxyethoxymethyl) guanine<br>3% myristyl alcohol<br>96.75% propylene glycol | 35 | 150 | 90 |
| 0.25% 9-(2-hydroxyethoxymethyl) guanine<br>3% oleic acid<br>96.75% propylene glycol | 100 | 320 | 280 |

The above data reflect relative transport of 9-(2-hydroxyethoxymethyl) guanine across human epidermis in vitro. Penetration is significantly enhanced by employing the binary system of this invention.

In the above skin penetration studies, human skin, (heat-separated abdominal epidermis, taken at autopsy), was placed in a standard Franz diffusion apparatus (Crown Glass Co., Somerville, N.J.) in a horizontal position between a lower, capped diffusion cell and an upper, open cell. A normal saline solution was added to the lower diffusion cell, abutting the subcutaneous side of the skin, and the test composition comprising a solution of active drug in the carrier at indicated formulation was added to the diffusion cell abutting the epidermal side of the skin.

This cell assembly was kept in a constant-temperature room at about 31°C. At appropriate intervals, each diffusion cell assembly was opened and the diffusate from the cell abutting the subcutaneous side of the skin was withdrawn. Drug active in the diffusate was measured using standard analytical techniques. Each trial was run on a separate sample of human skin.

The following are further nonlimiting examples of compositions of the present invention. All components are thoroughly mixed together by conventional formulating techniques to form the compositions described below.

EXAMPLE XIV

| | |
|---|---|
| 9-(2-hydroxyethoxymethyl) guanine | 0.5% |
| methyl laurate | 5.0% |
| propylene glycol | 25.0% |
| Carbopol 934P* | 0.6% |
| Polysorbate 80** | 0.2% |
| 1 N NaOH | 1.0% |
| Water | 67.7% |

EXAMPLE XV

| | |
|---|---|
| 9-(2-hydroxyethoxymethyl) guanine | 0.5% |
| oleic acid | 2.0% |
| propylene glycol | 25.0% |
| Carbopol 934P* | 0.6% |
| Polysorbate 80** | 0.2% |
| 1 N NaOH | 1.0% |
| Water | 70.7% |

EXAMPLE XVI

| | |
|---|---|
| 9-(2-hydroxyethoxymethyl) guanine | 0.5% |
| oleic acid | 4.0% |
| propylene glycol | 25.0% |
| Carbopol 934P* | 0.6% |
| Polysorbate 80** | 0.2% |
| 1 N NaOH | 1.0% |
| Water | 68.7% |

EXAMPLE XVII

| | |
|---|---|
| 9-(2-hydroxyethoxymethyl) guanine | 0.5% |
| oleic acid | 5.0% |
| propylene glycol | 94.5% |

* Carbopol 934P is a carboxyl vinyl polymer manufactured by the B.F. Goodrich Chemical Co.

** Polysorbate 80 is a surfactant, an oleate ester of sorbitol and its anhydrides copolymerized with approximately 20 moles of ethylene oxide for each mole of sorbitol or sorbitol anhydride.

0095813

CLAIMS

1. A composition of matter for topical administration, characterized in that it comprises:

(a) from 0.01% to 10 % by weight of substituted purine antiviral of the formula

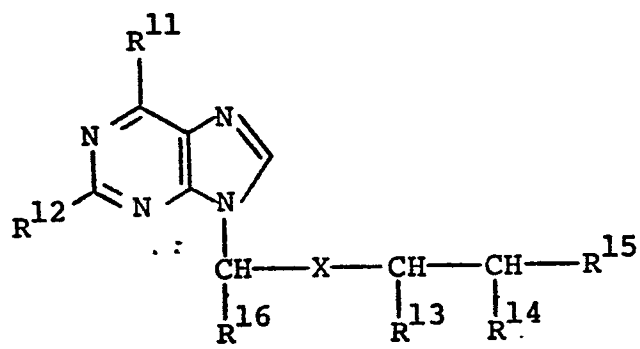

wherein X is sulfur or oxygen, $R^{11}$ is hydroxy, $R^{12}$ is amino, $R^{13}$ is hydrogen, a straight or branched chain or cyclic alkyl, hydroxyalkyl, benzyloxyalkyl or phenyl, $R^{14}$ is hydrogen, hydroxy or lower alkyl, $R^{15}$ is hydrogen, hydroxy amino, alkyl, hydroxyalkyl, benzoyloxy, benzoyloxy alkyl, benzyloxy, sulfamoyloxy, phosphate, carboxypropionyloxy, or acetoxy, $R^{16}$ is hydrogen, alkyl or hydroxyalkyl, or a pharmaceutically-acceptable salt thereof;

(b) from 0.0 to 70% of ethanol or 2-propanol;

(c) from about 0.01% to 99% by weight of a penetration-enhancing carrier consisting essentially of

(i) a diol compound selected from the group consisting of 1,2 ethanediol, 1,2-propanediol, 1,3-propanediol, 1,2- butanediol, 1,3- butanediol, 1,4-butanediol, 2,3-butanediol, 2-ethoxyethanol, 2,2-oxy-bis-ethanol, glycerol monoacetate, and mixtures thereof, and

(ii) a cell-envelope disordering compound of the formula

R'-X

where R' is a straight chain alkyl of about 7 to about 16 carbon atoms, a branched chain alkyl having from about 13 to about 22 carbon atoms, or a non-terminal alkenyl having from about 7 to

about 22 carbon atoms, X is $-OH$, $-COOH$, $-COOR^2$, $-O-\overset{O}{\overset{\|}{C}}-R^3$, $-\underset{R^5}{\underset{|}{CON}}-R^4$, $-COOC_2H_4OC_2H_4OH$, $-COOCH(CHOH)_4CH_2OH$, $-COOCH_2CHOHCH_3$, $-COOCH_2CHOHCH_2OH$, $-(OCH_2CH_2)_mOH$, $-SOCH_3$, or $-P(OCH_3)_2O$, and $R^2$ is $-H$, $-CH_3$, $-C_2H_5$, $-C_3H_7$, or $-C_2H_4OH$, $R^3$ is $-H$, $-CH_3$, $-C_2H_5$, $-C_3H_7$ or $C_2H_4OH$, $R^4$ is $-H$, $-CH_3$, $-C_2H_5$, $-C_3H_7$ or $-C_2H_4OH$, $R^5$ is $-H$, $-CH_3$, $-C_2H_5$, $-C_3H_7$ or $-C_2H_4OH$, and m is an integer from 2-4; provided that when R' is an alkenyl and X is -OH, at least one double bond is in the cis-configuration; the ratio of diol compound:cell-envelope disordering compound being in the range of 1:1 to 500:1 by weight.

2. A composition according to Claim 1 characterized in that the substituted purine antiviral is selected from the group consisting of 9-(2-hydroxyethoxymethyl) guanine, 2-amino-9-(2-hydroxyethoxymethyl)adenine, and pharmaceutically-acceptable salts thereof.

3. A composition according to Claim 1 or Claim 2 characterized in that the diol compound is selected from the group consisting of 1,2-propanediol 2,2-oxy-bis-ethanol, and mixtures thereof.

4. A composition according to any of Claims 1-3 characterized in that the cell-envelope disordering compound is selected from the group consisting of oleic acid, decenoic acid, dodecenoic acid, tetradecenoic acid, hexadecenoic acid, heptadecenoic acid, eicosenoic acid, docosenoic acid, oleyl alcohol, decenyl alcohol, dodecenyl alcohol, tetradecenyl alcohol, hexadecenyl alcohol, heptadecenyl alcohol, eicosenyl alcohol, docosenyl alcohol, methyl decanoate, methyl dodecanote, methyl tetradecanoate, ethyldecanoate, ethyl dodecanoate, ethyl tetradecanoate, propyl decanoate, propyl dodecanoate, propyl tetradecanoate, isopropyl decanoate, isopropyl dodecanote, isopropyl tetradecanoate, octyl acetate, decyl acetate, dodecanyl acetate, tetradecanyl acetate, and mixtures thereof.

5. A composition according to any of Claims 1-4 characterized in that the ratio of diol compound:cell-envelope disordering compound is in the range of from 10:1 to 100:1.

6. A composition according to any of Claims 1-5 characterized in that the ratio of diol compound:cell-envelope disordering compound is from 10:1 to 50:1.

7. A composition according to any of Claims 1-5 characterized in that it further comprises from 0% to 15% water by weight.

8. A composition according to any of Claims 1-7 characterized in that it further comprises from 0% to 10% water by weight.

9. A composition according to any of Claims 1-8 characterized in that it is substantially water-free.

10. A composition of matter for topical administration, characterized in that it comprises:

(a) from 0.01% to 10% by weight of 9-(2-hydroxyethoxymethyl) guanine;

(b) from 0% to 70% by weight of ethanol or 2-propanol;

(c) from 0.01% to 99% by weight of a penetration-enhancing carrier consisting essentially of

(i) a diol compound selected from the group consisting of 1,2-propanediol, 2,2-oxy-bis-ethanol, and mixtures thereof, and

(ii) a cell-envelope disordering compound selected from the group consisting of oleic acid, myristyl alcohol, methyl myristate, methyl laurate, ethyl myristate, ethyl laurate, myristyl acetate, lauryl acetate, and mixtures thereof; the ratio of diol compound:cell-envelope disordering compound being in the range of from 1:1 to 500:1 by weight.